(19) Europäisches Patentamt
European Patent Office
Office européen des brevets

(11) **EP 0 882 451 B1**

(12) **EUROPEAN PATENT SPECIFICATION**

(45) Date of publication and mention
of the grant of the patent:
**12.05.2004 Bulletin 2004/20**

(51) Int Cl.[7]: **A61K 31/198**, A61K 38/05,
A61P 37/06

(21) Application number: **98110072.0**

(22) Date of filing: **03.06.1998**

(54) **Glycine for prevention or treatment of transplant rejection**

Glycin zur Vorbeugung oder Behandlung von Transplantatabstossungen

Glycine pour la prévention ou le traitement du rejet de greffes

(84) Designated Contracting States:
**AT BE CH CY DE DK ES FI FR GB GR IE IT LI LU MC NL PT SE**

(30) Priority: **05.06.1997 US 869398**

(43) Date of publication of application:
**09.12.1998 Bulletin 1998/50**

(73) Proprietor: **Novartis Nutrition AG**
**3001 Bern (CH)**

(72) Inventors:
• **Schneider, Heinz**
  **1792 Cordast (CH)**
• **Cook, Nigel Scott**
  **4207 Bretzwil (CH)**

(74) Representative: **Schubert Santana, Isabelle et al**
**Novartis AG,**
**Patent & Trademark Dept.,**
**Lichtstrasse 35**
**4002 Basel (CH)**

(56) References cited:
EP-A- 0 705 542          WO-A-96/25861
WO-A-98/04255          WO-A-98/04256

• ABDALLAH A.N. ET AL: 'Plasma cytokines in graft vs host disease and complications following bone marrow transplantation' HEMATOL. CELL THERAP. vol. 39, 1997, pages 27 - 32
• TURNER D.M. ET AL: 'A genetic marker of high TNF-alfa production in heart transplant recipients' TRANSPLANTATION vol. 60, no. 10, 27 November 1995, pages 1113 - 1117
• HEEMANN U.W. ET AL: 'Infections and reduced functioning kidney mass induce chronic rejection in rat kidney allografts' CLINICAL NEPHROLOGY vol. 46, no. 1, 1996, pages 34 - 38

EP 0 882 451 B1

**Description**

The present invention relates to a new use of glycine alone or in combination with other therapeutic agents.

It is known to use a medicament or nutritional formulation comprising glycine, alanine or serine as an active ingredient to minimize or prevent the metabolic effects of a wide range of disease states or conditions induced by elevated tumor necrosis factor (TNF) levels. WO 96 /25861 discloses the use of glycine to decrease elevated TNF level, i.e. level of TNF beyond level that mediates physiological homeostasis and local inflammation.

WO 98/04256 discloses that glycine is suitable for the prophylactic and/or therapeutic treatment of renal dysfunction induced by cyclosporins or ascomycins.

It has now been found that glycine is indicated to prevent or treat chronic graft rejection. Thus the invention provides:

1.1 A method of preventing or treating manifestations of chronic rejection in a recipient of organ or tissue transplant, e.g. heart, lung, combined heart-lung, trachea, liver, bowel, kidney or pancreatic transplants, comprising the step of administering to said recipient a therapeutically effective amount of glycine, in free form or in pharmaceutically acceptable salt form;

Chronic rejection is considered as a multifactorial process in which not only the immune reaction towards the graft but also the response of the blood vessel wall in the grafted organ to injury plays a role. The variant of chronic rejection with the worst prognosis is an arteriosclerosis-like alteration, also called transplant vasculopathy graft vessel disease. This vascular lesion is characterized by migration and proliferation of smooth muscle cells. It appears to progress also through repetitive endothelial injury induced amongst others by host antibody or antigen-antibody complexes, through intimal proliferation and thickening, smooth muscle cell hypertrophy repair, and finally to gradual luminal obliteration. Also so-called non-immunological factors like hypertension, hyperlipidemia, hypercholesterolemia etc. play a role.

Thus the invention further provides:

1.2 A method of preventing or treating vasculopathy graft vessel disease in a recipient of organ or tissue transplant.

In a series of further specific or alternative embodiments, the present invention also provides:

2.1 Use of glycine, in free form or in pharmaceutically acceptable salt form, in combination with an immunosuppressant or immunomodulating agent or a mixture thereof, in a method as defined hereinafter under 1.1 or 1.2;

2.2 Use of glycine, in free form or in pharmaceutically acceptable salt form, in the preparation of a medicament or nutritional formulation for a combined use with an immunosuppressant or immunomodulating agent or a mixture thereof, in a method as defined hereinafter under 1.1 or 1.2;

3.1 A method of preventing or treating vasculopathy graft vessel disease in a recipient of organ or tissue transplant, e.g. heart, lung, combined heart-lung, trachea, liver, bowel, kidney or pancreatic transplants, comprising the step of administering to said recipient glycine, in free form or in pharmaceutically acceptable salt form, and an immunosuppressant or immunomodulating agent or a mixture thereof, in a therapeutically effective amount;

3.2 A method of preventing or treating manifestations of chronic rejection in a recipient of organ or tissue transplant, e.g. heart, lung, combined heart-lung, trachea, liver, bowel, kidney or pancreatic transplants, comprising the step of administering to said recipient glycine, in free form or in pharmaceutically acceptable salt form, and an immunosuppressant or immunomodulating agent or a mixture thereof, in a therapeutically effective amount;

4.1 Use of a kit or package in any method as defined hereinabove, said kit or package including a pharmaceutical composition or nutritional formulation comprising glycine in free form or in pharmaceutically acceptable salt form and a pharmaceutical composition comprising an immunosuppressant or immunomodulating agent, together with instructions to use.

According to a preferred embodiment of the invention, glycine is indicated optionally in combination with an immunosuppressant or immunomodulating agent for preventing or treating manifestations of chronic rejection.

According to the invention, glycine is conveniently employed in the L-configuration, in free amino add form, in the form of glycine precursors, in particular alanine or serine, likewise in free amino acid form, in pharmaceutically acceptable salt form of said amino acids, or in form of mixtures of said amino adds and/or pharmaceutically acceptable salts thereof. Glycine is preferably used in free amino acid form, in pharmaceutically acceptable salt form or in the form of a mixture of glycine in free amino acid form with glycine in pharmaceutically acceptable salt form; most preferably glycine is in free amino acid form. Glycine may also be used in the form of a dipeptide according to the invention. Suitable salts include e.g. mineral or organic acid salts of the amino residue, and alkali or organic salts of the carboxylic acid residue.

Daily dosages of glycine required in treating or preventing diseases and conditions according to the invention will vary depending upon, for example, the host, the mode of administration, the severity of the condition to be treated and the optionally used (concomitantly, separately or in sequence) immunosuppressant or immunomodulating agent. A preferred daily dosage of glycine is 1 to 80g, preferably, 1 to 60g, particularly preferred 15 to 30g. It may also be administered to the patient in an amount such that its concentration in the patients' plasma is elevated to between 0.5 and 2.0 mM, preferably from 1.0 to 2.0 mM, although concentrations higher than this may be used. Suitable examples of immunosuppressants or immunomodulating agents include e.g. cyclosporins, rapamycins or ascomycins or their immunosuppressive analogs, e.g. Cyclosporin A, Cyclosporin G, FK-506, rapamycin, 40-O-(2-hydroxy)ethyl-rapamycin; corticosteroids; cyclophosphamide; azathioprine; methotrexate; brequinar; leflunomide; mizoribine; mycophenolic acid; mycophenolate mofetil; 15-deoxyspergualine; immunosuppressive antibodies, e.g. monoclonal antibodies to leukocyte receptors, e.g. MHC, CD2, CD3, CD4, CD7, CD25, CD28, B7, CD45, or CD 58 or their ligands; or other immunomodulatory compounds, e.e. CTLA4Ig.

Where glycine is administered in conjunction, i.e. sequentially, separately or concomitantly, with other immunosuppressive or immunomodulating therapy, e.g. as hereinabove specified, dosage of the co-administered immunosuppressant or immunomodulating agent will of course vary depending on the type of co-drug employed, e.g. whether it is a cyclosporin or an immunosuppressive antibody, on the specific drug employed, on the condition being treated, and so forth. According to the invention, glycine may also be administered in conjunction with a combination of immunosuppressants/immunomodulating agents, e.g. Cyclosporin A/ Steroids/Azathioprine or mycophenolate mofetil.

Glycine may be administered to the patient enterally or parenterally. The enteral administration route is preferred; particularly contemplated enteral administration routes are oral and/or tube feeding such as nasal feeding, particularly oral feeding. The medicament or nutritional formulation is conveniently administered in the form of an aqueous liquid. The medicament or nutritional formulation in a form suitable for enteral application is accordingly preferably aqueous or in powder form, whereby the powder is conveniently added to water prior to use. For use in tube feeding, the amount of water to be added will depend, inter alia, on the patients fluid requirements and condition. It will be appreciated that, for acute treatment, the parenteral application route is preferred.

When glycine is administered in the form of a medicament, it may be used together with pharmaceutically acceptable diluents or carriers, e.g. such for enteral or parenteral administration.

When glycine is administered in the form of a nutritional formulation, it may be used in combination with one or more of the following components:

omega-3 polyunsaturated fatty acids, L-arginine or L-ornithine, a nucleobase source, e.g. a formulation as disclosed in US Ser. Nr.08/690476.

Glycine may also be administered in the form of a nutritional formulation which is a formula diet, e.g. a complete formula diet or preferably a diet supplement which can be administered over a long period of time. According to the invention formula diets may comprise, in addition to glycine, a source of carbohydrates, lipids fat (fat source), protein (nitrogen source) and optionally further nutritional components such as vitamins, minerals, trace elements and/or fibers (preferably soluble fibers), e.g. a formula diet as disclosed in US Ser. Nr.08/690476. Preferably diet supplements for chronic administration comprise, in addition to glycine, a source of carbohydrates and further nutritional components such as vitamins, minerals, trace elements and flavoring agents and optionally arginine.

The medicament, diets and formulations of the invention may be obtained in a manner known per se, e.g. by admixing the ingredients.

Utility of glycine in treating or preventing diseases and conditions as hereinabove specified may be demonstrated in in vitro and in animal tests, for example with the methods described in the Examples.

The nutritional formulations of the invention are further illustrated by the Examples.

EXAMPLE 1: Nutritional Formulations

| MM (Mineral Mix) | |
|---|---|
| Ingredients | g/100g |
| Maltodextrins | 34.40 |
| K citrate/phosphate | 34.60 |
| Magnesium dicitrate | 8.20 |

(continued)

| MM (Mineral Mix) | |
|---|---|
| Ingredients | g/100g |
| Calcium chloride | 8.00 |
| Sodium citrate/chloride | 9.00 |
| Citric acid | 3.50 |
| Choline tartrate | 2.30 |

| SM (Trace Elements Mix) | |
|---|---|
| Ingredients | g/100g |
| Maltodextrins | 47.79 |
| Molybdenum-yeast | 18.00 |
| Chromium-yeast | 9.20 |
| Zinc sulfate | 7.00 |
| Selenium-yeast | 7.00 |
| Ferrum(II) sulfate | 6.92 |
| Copper(II) gluconate | 2.24 |
| Manganese(II) sulfate | 1.12 |
| Sodium fluoride | 0.70 |
| Potassium iodide | 0.03 |

| Composition I Comprising Glycine | |
|---|---|
| Ingredients | g/100g |
| Water | 77.40 |
| Maltodextrins | 12.28 |
| Na/Ca caseinates | 4.60 |
| Glycine | 3.00 |
| Lipids: | |
| Palm oil | 2.33 |
| Sunflower oil | 0.26 |
| Emulsifier Nathin E | 0.13 |
| | 100.00 |

| VM (Vitamin Mix) | |
|---|---|
| Ingredients | g/100g |
| Maltodextrins | 43.44 |
| Sodium ascorbate | 35.00 |
| Vitamin E-Ac. 50% | 16.00 |
| Niacinamide | 1.55 |
| Vitamin A-Acetate | 1.20 |
| Ca-D-Panthothenat | 0.98 |
| Vitamin $K_1$ 1% | 0.71 |
| Vitamin $B_{12}$ 0.1% | 0.30 |
| Vitamin $D_3$ | 0.28 |
| Vitamin $B_6$ | 0.20 |
| Vitamin $B_1$ | 0.17 |

(continued)

| VM (Vitamin Mix) | |
|---|---|
| Ingredients | g/100g |
| Vitamin $B_2$ | 0.15 |
| Folic acid | 0.02 |
| Biotin | 0.01 |

| Composition II Comprising Glycine | |
|---|---|
| Ingredients | g/100g |
| Water | 77.40 |
| Maltodextrins | 10.10 |
| Na/Ca caseinates | 4.60 |
| Glycine | 3.00 |
| Mineral Mix | 2.00 |
| Trace Elements Mix | 0.05 |
| Vitamin Mix | 0.10 |
| β-Carotine | 0.03 |
| Lipids: | |
| Palm oil | 2.33 |
| Sunflower oil | 0.26 |
| Emulsifier Nathin E | 0.13 |
| | 100.00 |

| Composition III Comprising Glycine (in powder form) | |
|---|---|
| Ingredients | g/100g |
| Sugar | 59.163 |
| Glycine | 33.333 |
| Sodium citrate/chloride | 3.333 |
| Citric acid | 2.889 |
| Potassium chloride | 0.444 |
| Fresh flavour | 0.307 |
| Maltodextrins | 0.296 |
| Vitamin C | 0.129 |
| Magnesium carbonate | 0.053 |
| Calcium phosphate | 0.033 |
| Vitamin E-Ac. 50% | 2.000 |
| | 100.000 |

| Composition I Comprising Glycine and Arginine | |
|---|---|
| Ingredients | g/100g |
| Water | 77.40 |
| Maltodaxtrins | 8.93 |
| Na/Ca caseinates | 4.60 |
| Glycine | 3.00 |
| Arginine | 1.17 |
| Mineral Mix | 2.00 |

(continued)

| Composition I Comprising Glycine and Arginine | |
|---|---|
| Ingredients | g/100g |
| Trace Elements Mix | 0.05 |
| Vitamin Mix | 0.10 |
| β-Carotine | 0.03 |
| Lipids: | |
| Palm oil | 2.36 |
| Sunflower oil | 0.23 |
| Emulsifier Nathin E | 0.13 |
| | 100.00 |

| Composition II Comprising Glycine and Arginine (in powder form) | |
|---|---|
| Ingredients | g/100g |
| Sugar | 59.937 |
| Glycine | 25.000 |
| Arginine | 15.833 |
| Sodium citrate/chloride | 2.499 |
| Citric acid | 1.667 |
| Potassium chloride | 0.333 |
| Fresh flavour | 0.333 |
| Maltodextrins | 0.221 |
| Vitamin C | 0.097 |
| Magnesium carbonate | 4.000 |
| Calcium phosphate | 2.500 |
| Vitamin E-Ac. 50% | 1.500 |
| | 100.000 |

| Composition Comprising Glycine and Fish Oil ($\omega$-3 fatty acids) | |
|---|---|
| Ingredients | g/100g |
| Water | 77.40 |
| Maltodextrins | 10.10 |
| Na/Ca caseinates | 4.60 |
| Glycine | 3.00 |
| Mineral Mix | 2.00 |
| Trace Elements Mix | 0.05 |
| Vitamin Mix | 0.10 |
| β-Carotine | 0.03 |
| Lipids: | |
| Palm oil | 1.32 |
| Sunflower oil | 0.23 |
| Emulsifier Nathin E | 0.13 |
| Fish Oil EPAX 3000 TG | 1.04 |
| | 100.00 |

EXAMPLE 2: The Effect of Dietary Glycine on Chronic Rejection in a Rat Aortic Transplant Model

Orthotopic aortic transplantation between F-344 rats (donor) and Lewis rats (recipient) are completed across minor histocompatibility barriers. Recipients are fed control diet or glycine-containing diet (5%, w/w) for three days before transplantation. Rats are maintained on the same diets until sacrifice 6 or 8 weeks after transplantation. Grafts are removed and a portion is frozen or fixed in formalin for evaluation. Area of the four regions of the cross section of the aorta (lumen, intima, media and adventitia) are determined for each allograft. In cross sections of aortic allografts, intima proliferation (thickening), media necrosis (thinning) and infiltration of the adventitia (perivascular inflammation) are observed in all grafts regardless of diet. These features are typical of chronic rejection in the aortic transplant model, comparable to graft arteriosclerosis in human transplants. Aortic allografts from glycine fed recipients have decreased medial necrosis (in allografts of rats fed control diet, the medial area is reduced significantly to $1.5 \pm 0.3$ x $10^5$ $\mu m^2$, $p < 0.05$, in contrast to medial areas of allografts from glycine fed rats, $3.5 \pm 0.2$ x $10^5$ $\mu m^2$, $p < 0.05$, or medial areas of untransplanted abdominal aortas of Fisher-344 rats $3.3 \pm 0.3$ x $10^5$ $\mu m^2$, $p < 0.05$) and a reduced perivascular inflammation (reduced number of infiltrating leukocytes in allograft adventita: $202 \pm 18$ vs. $140 \pm 13$, $p < 0.05$). Dietary glycine increased the luminal area of a transplanted aorta significantly to $5.9 \pm 0.7$ vs. $3.6 \pm 0.2$ x $10^5$ $\mu m^2$, $p < 0.05$ in comparison to a non-transplanted aorta. In addition, dietary glycine significantly reduced the ratio of intimal to medial area in the transplanted aorta to $0.45 \pm 0.15$ vs. $1.75 \pm 0.4$, $p < 0.05$ the recipients receiving control diet.

## Claims

1. Use of glycine, glycine dipeptide, alanine, serine, and/or mixture thereof in the preparation of a medicament or nutritional formulation for preventing or treating manifestations of chronic rejection in a recipient of organ or tissue transplant.

2. Use of glycine, glycine dipeptide, alanine, serine and/or mixture thereof in the preparation of a medicament or nutritional formulation for preventing or treating vasculopathy graft vessel disease in a recipient of organ or tissue transplant.

3. The use according to any one of claims 1 to 2 wherein the medicament or nutritional formulation comprising glycine, glycine dipeptide, alanine, serine and/or mixture thereof is indicated for concomitant, separate or sequential administration with an immunosuppressant or an immunomodulating agent or a mixture thereof.

4. The use of any one of claims 1 to 3, wherein the medicament or nutritional formulation comprising glycine, glycine dipeptide, alanine, serine and/or mixture thereof is provided together with a pharmaceutical composition comprising an immunosuppressant or immunomodulating agent in form of a kit or package.

5. The use according to claim 3 or 4, wherein said immunosuppressant or immunomodulating agent is selected from the group consisting of cyclosporins, rapamycins, ascomycins, corticosteroids, cyclophosphamide, azathioprine, methotrexate, brequinar, leflunomide, mizoribine, mycophenolic acid, mycophenolate mofetil, 15-deoxyspergualine, immunosuppressive antibodies, ligands thereof and CTLA4Ig.

6. The use according to claim 5, wherein said immunosuppressant or immunomodulating agent is selected from the group consisting of Cyclosporin A, Cyclosporin G, FK-506, rapamycin, 40-O-(2-hydroxy)ethyl-rapamycin, corticosteroids, azathioprine, mycophenolic acid and mycophenolate mofetil.

## Patentansprüche

1. Verwendung von Glycin, Glycindipeptid, Alanin, Serin und/oder einem Gemisch davon bei der Herstellung eines Arzneimittels oder einer Nährmittelformulierung zur Prävention oder Behandlung der Erscheinungsformen von chronischer Wiederabstoßung bei einem Rezipienten von Organoder Gewebstransplantat.

2. Verwendung von Glycin, Glycindipeptid, Alanin, Serin und/oder einem Gemisch davon bei der Herstellung eines Arzneimittels oder einer Nährmittelformulierung zur Prävention oder Behandlung einer Vaskulopathietransplantatgefäßerkrankung bei einem Rezipienten eines Organ- oder Gewebstransplantats.

3. Verwendung nach einem beliebigen der Ansprüche 1 bis 2, wobei das Arzneimittel oder die Nährmittelformulierung, umfassend Glycin, Glycindipeptid, Alanin, Serin und/oder ein Gemisch davon, zur gleichzeitigen, getrennten oder aufeinander folgenden Verabreichung mit einem Immunosuppressant oder einem immunomodulierenden Mittel oder einem Gemisch davon angezeigt ist.

**4.** Verwendung nach einem beliebigen der Ansprüche 1 bis 3, wobei das Arzneimittel oder die Nährmittelformulierung, umfassend Glycin, Glycindipeptid, Alanin, Serin und/oder ein Gemisch davon, zusammen mit einer pharmazeutischen Zusammensetzung, umfassend ein Immunosuppressant oder immunomodulierendes Mittel, in Form eines Kits oder einer Packung bereitgestellt wird.

**5.** Verwendung nach Anspruch 3 oder 4, wobei das Immunosuppressant oder immunomodulierende Mittel aus der Gruppe bestehend aus Cyclosporinen, Rapamycinen, Ascomycinen, Corticosteroiden, Cyclophosphamid, Azathioprin, Methotrexat, Brequinar, Leflunomid, Mizoribin, Mycophenolsäure, Mycophenolatmofetil, 15-Deoxyspergualin, immunosuppressiven Antikörpern, Liganden davon und CTLA4lg, ausgewählt ist.

**6.** Verwendung nach Anspruch 5, wobei das Immunosuppressant oder immunomodulierende Mittel aus der Gruppe bestehend aus Cyclosporin A, Cyclosporin G, FK-506, Rapamycin, 40-0-(2-Hydroxy)ethyl-rapamycin, Corticosteroiden, Azathioprin, Mycophenolsäure und Mycophenolatmofetil, ausgewählt ist.

**Revendications**

**1.** Utilisation de glycine, de dipeptide de glycine, d'alanine, de sérine et/ou d'un mélange de ceux-ci, dans la préparation d'un médicament ou d'une formulation nutritionnelle pour la prévention ou le traitement de manifestations de rejet chronique chez le receveur d'une greffe d'un organe ou tissu.

**2.** Utilisation de glycine, de dipeptide de glycine, d'alanine, de sérine et/ou d'un mélange de ceux-ci, dans la préparation d'un médicament ou d'une formulation nutritionnelle pour la prévention ou le traitement d'une maladie vasculaire de greffe de vasculopathie chez le receveur d'une greffe d'un organe ou tissu.

**3.** Utilisation suivant l'une quelconque des revendications 1 et 2, dans laquelle le médicament ou la formulation nutritionnelle comprenant de la glycine, du dipeptide de glycine, de l'alanine, de la sérine et/ou un mélange de ceux-ci, est indiqué(e) pour une administration concomitante, séparée ou séquentielle avec un immunosuppresseur ou un agent immunomodulateur, ou un mélange de ceux-ci.

**4.** Utilisation suivant l'une quelconque des revendications 1 à 3, dans laquelle le médicament ou la formulation nutritionnelle comprenant de la glycine, du dipeptide de glycine, de l'alanine, de la sérine et/ou un mélange de ceux-ci est fourni(e) conjointement avec une composition pharmaceutique comprenant un immunosuppresseur ou un agent immunomodulateur sous forme d'une trousse ou d'un ensemble.

**5.** Utilisation suivant la revendication 3 ou 4, dans laquelle ledit immunosuppresseur ou agent immunomodulateur est choisi dans le groupe constitué par des cyclosporines, des rapamycines, des ascomycines, des corticostéroïdes, le cyclophosphamide, l'azathioprine, le méthotrexate, le bréquinar, le léflunomide, la mizoribine, l'acide mycophénolique, le mycophénolate mofétil ; la 15-désoxyspergualine, des anticorps immunosuppresseurs, leurs ligands, et CTLA4lg.

**6.** Utilisation suivant la revendication 5, dans laquelle ledit immunosuppresseur ou agent immunomodulateur est choisi dans le groupe constitué par la Cyclosporine A, la Cyclosporine G, FK-506, la rapamycine, la 40-O-(2-hydroxy)éthyl-rapamycine, des corticostéroïdes, l'azathioprine, l'acide mycophénolique et le mycophénolate mofétil.